# EUROPEAN PATENT SPECIFICATION

(11) **EP 2 617 410 B1**
(45) Date of publication and mention of the grant of the patent: **27.12.2017**
(21) Application number: 11824923.4
(22) Date of filing: 10.08.2011
(51) Int. Cl.: A61K 8/81, A61K 8/06, A61Q 19/00, A61K 8/49

(54) **OIL-IN-WATER TYPE COSMETIC**
ÖL-IN-WASSER-KOSMETIKUM
PRODUIT COSMÉTIQUE DE TYPE ÉMULSION HUILE DANS L'EAU

(30) Priority: 15.09.2010 JP 2010207308
(43) Date of publication of application: 24.07.2013
(73) Proprietor: FUJIFILM Corporation, Tokyo 106-8620 (JP)
(72) Inventor: KINAI, Miki, Ashigarakami-gun Kanagawa 258-8577 (JP)
(74) Representative: Klunker IP Patentanwälte PartG mbB
(86) International application number: PCT/JP2011/068315
(87) International publication number: WO 2012/035915

(56) References cited:
- JP-A- 2005 089 303
- JP-A- 2007 077 096
- JP-A- 2008 050 271
- JP-A- 2009 242 269
- JP-A- 2009 242 269
- JP-A- 2011 111 402
- US-A1- 2007 207 103
- DATABASE GNPD [Online] MINTEL; 3 March 2008 (2008-03-03), "White Lifting Liquid", XP002745483, Database accession no. 899929
- DATABASE GNPD [Online] MINTEL; 1 February 2007 (2007-02-01), "Body Firming Moisturizer", XP002745484, Database accession no. 659572
- DATABASE GNPD [Online] MINTEL; 1 October 2015 (2015-10-01), "Moist Lifting Liquid", XP002745485, Database accession no. 798324

## Description

### Technical Field

The present invention relates to an oil-in-water cosmetic.

### Background Art

Various kinds of technologies have been developed with the aim of imparting resilient feeling to a skin.

For example, Japanese Patent Application Laid-Open (JP-A) No. 2000-239139 discloses a skin cosmetic including a film forming agent such as: polyvinyl alcohol, a latex, a cellulose derivative, or a silicone type resin; a lower alcohol; a spherical powder; and an algefacient, to form a cosmetic film having an adequate thickness on a skin. Further, JP-A No. 2009-234971 discloses an oil-in-water cosmetic in which a specified solid oil such as polyethylene wax or beeswax is combined with a specified sucrose fatty acid ester and a polyglycerin fatty acid ester.

Moreover, JP-A No. 2007-261971 discloses an oil-in-water emulsified cosmetic that is capable of imparting resilient feeling by using a combination of a highly viscous oil, isotridecyl isononanoate, and a polymer such as polyethylene glycol, instead of a solid oil and a film-forming agent.

### SUMMARY OF INVENTION

### Technical Problem

However, when a film-forming agent is used to impart resilient feeling, there is a case in which sticky feeling is imparted depending on the amount of the film-forming agent contained. In a cosmetic containing a large amount of a solid oil, there has been a case in which aggregation and coalescence of emulsification particles, crystal precipitation, or the like occurs to cause a problem in terms of stability over time.

Even when a combination of a high viscosity oil and a polymer is used in a cosmetic instead of a film-forming agent and a solid oil, there is a case in which sticky feeling is imparted due to the polymer.

As described above, there still has been a demand for an oil-in-water cosmetic capable of imparting good feeling of use, particularly resilient feeling, as well as having excellent stability over time.

Therefore, the present invention provides an oil-in-water cosmetic capable of imparting good feeling of use, particularly resilient feeling, as well as having excellent stability over time.

### Solution to Problem

In the following, the present invention is described.
[1] An oil-in-water cosmetic including:
   an aqueous phase; and
   an oil phase including a hydrogenated polyisobutene having a kinematic viscosity at 100 °C of from 200 mm²/s to 1000 mm²/s and sorbitan stearate,
   wherein the kinematic viscosity is measured by the method described in JIS-K-2283, and
   wherein the oil phase further comprises both a higher alcohol and a higher fatty acid, the higher alcohol being an alcohol having from 12 to 24 carbon atoms, and the higher fatty acid being a saturated or unsaturated fatty acid having from 12 to 24 carbon atoms.
[2] The oil-in-water cosmetic according to [1], wherein a total content of a solid oil, which has a melting point of at least 60°C and is selected from an oil, a fat, a hydrocarbon, or a wax, is 0 or less than 5% by mass, with respect to a total mass of the cosmetic.
[3] The oil-in-water cosmetic according to [1] or [2], wherein a content of the oil phase is from 45% by mass to 70% by mass with respect to a total mass of the cosmetic.
[4] The oil-in-water cosmetic according to any one of [1] to [3], wherein a content of the hydrogenated polyisobutene is from 10% by mass to 40% by mass with respect to the mass of the oil phase.
[5] The oil-in-water cosmetic according to any one of [1] to [4], wherein a content of the hydrogenated polyisobutene is from 5% by mass to 20% by mass with respect to a total mass of the cosmetic.
[6] The oil-in-water cosmetic according to [6] or [7], wherein the higher alcohol is at least one selected from behenyl alcohol or stearyl alcohol.
[7] The oil-in-water cosmetic according to any one of [6] to [8], wherein the higher fatty acid is at least one selected from palmitic acid or stearic acid.

### DESCRIPTION OF EMBODIMENTS

The oil-in-water cosmetic according to the invention is an oil-in-water cosmetic including: an aqueous phase; and an oil phase including a hydrogenated polyisobutene having a kinematic viscosity at 100 °C of from 200 mm²/s to 1000 mm²/s and sorbitan stearate.

According to the invention, good feeling of use, in particular, resilient feeling, is imparted by a combination of a specific hydrogenated polyisobutene having a high viscosity and sorbitan stearate. Accordingly, good feeling of use can be obtained without using a polymer compound such as polyethylene glycol. Further, occurrence of separation or precipitation is suppressed because it is not necessary to use a solid oil or a film-forming agent.

Hereinafter, the invention is described.

The term "process" in the present specification encompasses an independent process, as well as a process that cannot be clearly distinguished from another process but yet achieves the expected effect of the process of interest.

In the present invention, any numerical range expressed herein using "to" refers to a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

The oil-in-water cosmetic according to the invention is an oil-in-water emulsified composition including an oil phase and an aqueous phase, and the oil phase is dispersed in the aqueous phase as oil droplets (oil-in-water emulsified particles).

The oil-in-water cosmetic according to the invention includes a hydrogenated polyisobutene having a kinematic viscosity at 100 °C of from 200 mm²/s to 1000 mm²/s as an ingredient for an oil phase.

The hydrogenated polyisobutene has a kinematic viscosity at 100 °C of from 200 mm²/s to 1000 mm²/s. When the hydrogenated polyisobutene having a kinematic viscosity of more than 1000 mm²/s, has too high viscosity and does not provide good feeling of use, as a result of which there is a case in which stability deteriorates. In contrast, when a hydrogenated polyisobutene has a kinematic viscosity of less than 200 mm²/s, good feeling of use cannot be obtained since the viscosity is too low, as a result of which effects of the invention cannot be expected. From the viewpoints of feeling of use and stability over time, the hydrogenated polyisobutene having a kinematic viscosity at 100 °C of from 300 mm²/s to 800 mm²/s is more preferable. The kinematic viscosity in the invention is measured based on Crude Petroleum and Petroleum Product--Determination of Kinematic Viscosity and Calculation of Viscosity Index described in JIS-K-2283.

The hydrogenated polyisobutene may be, for example, one selected from the group consisting of a heavy liquid isoparaffin and a hydrogenated polyisobutene, based on the kinematic viscosity. The hydrogenated polyisobutene is also available as a commercial product, and examples thereof include PARLEAM 18 (kinematic viscosity of 300 mm²/s) and PARLEAM 24 (Kinematic viscosity of 800 mm²/s), both manufactured by NOF Corporation. These may be used singly, or in combination of two or more kinds thereof.

The content of the hydrogenated polyisobutene in the oil-in-water cosmetic according to the invention is preferably from 5 % by mass to 20 % by mass, more preferably from 8 % by mass to 15 % by mass, and further preferably from 9 % by mass to 12 % by mass, with respect to the total mass of the cosmetic, from the viewpoint of imparting good feeling of use, in particular, resilient feeling.

The content of the hydrogenated polyisobutene is preferably from 10 % by mass to 40 % by mass, more preferably from 16 % by mass to 30 % by mass, and further preferably from 18 % by mass to 23 % by mass, with respect to the mass of the oil phase in the oil-in-water cosmetic, from the viewpoint of imparting good feeling of use, in particular, resilient feeling.

The oil phase in the oil-in-water cosmetic in the invention means total components added as an oil phase in the production of the oil-in-water emulsified composition, and specifically means, mainly, oils (such as liquid oils, semi-solid oils, and solid oils), surfactants (liquid surfactants, semi-solid surfactants, and solid surfactants), particular higher alcohols, and particular higher fatty acids. In addition, oil-soluble colorants and oil-soluble proteins, etc. are also encompassed. Further, a mixture thereof such as a variety of vegetable oils and animal oils are also included. Substances included in each compound type may be used singly, or in combination of two or more kinds thereof. Further, oil-soluble preservatives, ultraviolet absorbing agents, oil-soluble drugs, antioxidants, and perfumes can be included as an oil phase. Substances included in each compound type may be used singly, or in combination of two or more kinds thereof. Accordingly, the oil phase in the oil-in-water cosmetic according to the invention may include variety of oil-soluble substances described below as ingredients for an oil phase, in addition to the hydrogenated polyisobutene.

In particular, the oil-in-water cosmetic according to the invention includes a higher alcohol and a higher fatty acid, as ingredients for an oil phase so as to allow the effect in terms of improvement of feeling of use to persist, and also the oil-in-water cosmetic according to the invention includes both a higher alcohol and a higher fatty acid, from the viewpoints of feeling of use, in particular, resilient feeling and moisturizing feeling; and persistence thereof.

The higher alcohol is a higher alcohol having from 12 to 24 carbon atoms, and examples thereof include a higher alcohol such as behenyl alcohol, stearyl alcohol, isostearyl alcohol, cetanol, or 2-octyl dodecanol, and at least one selected from the group consisting of behenyl alcohol and stearyl alcohol may be preferably used. These may be used singly, or in combination of two or more thereof.

The content of the higher alcohol in the oil-in-water cosmetic is preferably from 0.5 % by mass to 5 % by mass, and more preferably from 1 % by mass to 4 % by mass, with respect to the total mass of the cosmetic, from the viewpoints of feeling of use, in particular, resilient feeling and moisturizing feeling, and persistence thereof.

The higher fatty acid is a saturated or unsaturated fatty acid having from 12 to 24 carbon atoms, and examples thereof include myristic acid, palmitic acid, stearic acid, isostearic acid, linolic acid, and arachidonic acid. At least one selected from the group consisting of palmitic acid and stearic acid may be preferably used. These may be used singly, or in combination of two or more kinds thereof.

The content of the higher fatty acid in the oil-in-water cosmetic is preferably from 0.5% by mass to 5% by mass, and more preferably from 1% by mass to 4 % by mass, with respect to the total mass of the cosmetic, from the viewpoints of feeling of use, in particular, resilient feeling and moisturizing feeling, and persistence thereof.

The combination of the higher alcohol and the higher fatty acid is not particularly limited, however, preferable examples thereof include a combination of at least one kind of higher alcohol selected from the group consisting of behenyl alcohol and stearyl alcohol and at least one kind of higher fatty acid selected from the group consisting of palmitic acid and stearic acid; and a combination of stearic acid and stearyl alcohol or a combination of palmitic acid and behenyl alcohol is more preferable.

The oil-in-water cosmetic according to the invention may include oils and fats, waxes, hydrocarbons, or the like as an oil content in the oil phase component.

Examples of oils and fats include hardened coconut oil, hardened castor oil, palm oil, hardened oil, and Japan wax.

Examples of waxes include candelilla wax having a melting point of from 68°C to 72°C, carnauba wax having a melting point of from 80°C to 86°C, beeswax having a melting point of from 60°C to 67°C, montan wax, rice bran wax, sugar cane wax, and palm wax.

Examples of hydrocarbons include vaseline, ceresin, paraffin wax, microcrystalline wax, polyethylene wax, squalane, hydrogenated polydecene, and liquid paraffin.

In the invention, among the oils and fats, hydrocarbons, and waxes described above, the total content of the solid oil having a melting point of 60°C or more is preferably 0, or less than 5% by mass, with respect to the total mass of the cosmetic, from the viewpoint of stability over time.

The solid oil having a melting point of 60°C or more that is selected from the group consisting of oils and fats, hydrocarbons and waxes is selected based on the melting points of the individual substances. For example, candelilla wax, carnauba wax, and beeswax correspond to solid oils having a melting point of 60°C or more, vaseline and liquid paraffin do not correspond to solid oils having a melting point of 60°C or more.

Examples of other oils includes, for example, sterols such as cholesterol and phytosterol; and esters such as ethyl hexyl palmitate, isopropyl myristate, and octyl dodecyl myristate.

As functional oils having a specific function, carotenoids such as β-carotene, as-taxanthin, zeaxanthin, lycopene, and lutein; vitamin Es such as tocopherol and tocotrienol; ubiquinones such as coenzyme Q10; and ω-3 fatty acids such as eicosapentaenoic acid (EPA), docosahexaenoic acid (DHA), and linolenic acid may further be included.

The term "functional component" in the present specification means a component that is expected to induce specified physiological effects, when the component is applied to a living body.

Further, although it is costly, active ceramides such as ceramide I, ceramide II, ceramide III, ceramide V, and ceramide VI; glycosphingolipids such as glucosylceramide and galactosylceramide; sphingomyelins; and pseudoceramides may be included as oils having a moisturizing function.

These ingredients for an oil phase other than the hydrogenated polyisobutene may be contained to the extent that the function by the specific hydrogenated polyisobutene according to the invention is not impaired.

The oil-in-water cosmetic according to the invention includes sorbitan stearate as an ingredient for the oil phase in addition to the above-described specific hydrogenated polyisobutene. It is thought that oil droplets that are easily broken apart under moderate pressure are formed by combining sorbitan stearate with the specific hydrogenated polyisobutene described above, whereby a favorable feeling when used is imparted together with the hydrogenated polyisobutene.

The content of the sorbitan stearate in the oil-in-water cosmetic is preferably from 0.3% by mass to 2% by mass, and more preferably from 0.5% by mass to 1.0% by mass, with respect to the total mass of the cosmetic, from the viewpoint of use sensation (in particular, the sense of resilience).

The mass of the specific hydrogenated polyisobutene described above is preferably from 5 times to 25 times, and more preferably from 9 times to 20 times, the mass of the sorbitan stearate, from the viewpoint of use sensation.

The oil-in-water cosmetic according to the invention may contain known surfactants as ingredients for an oil phase in addition to the hydrogenated polyisobutene and sorbitan stearate, to the extent that the effect of the invention is not impaired. Examples of surfactants include anionic, cationic, amphoteric, and nonionic surfactants. In particular, nonionic surfactants are preferable, and examples thereof include polyglycerol fatty acid esters, sucrose fatty acid esters, glycerol fatty acid esters, organic acids of glycerol fatty acid esters, sorbitan fatty acid esters other than sorbitan stearate, propylene glycol fatty acid esters, polysorbates and combinations thereof.

The oil-in-water cosmetic according to the invention may include oil-soluble preservatives, ultraviolet absorbing agents, oil-soluble drugs, antioxidants, and the like as ingredients for an oil phase, in addition to the ingredients described above.

The mass of the oil phase as the total of ingredients for an oil phase in the oil-in-water cosmetic is preferably from 45 % by mass to 70 % by mass, and more preferably from 47 % by mass to 52 % by mass, with respect to the total mass of the cosmetic, from the viewpoints of feeling of use and stability.

Addition of water as an aqueous phase in the present oil-in-water cosmetic is necessary. The content of water is the remaining portion other than non-water components and is approximately from 10 % by mass to 50 % by mass.

The present oil-in-water cosmetic may include a polyhydric alcohol as an ingredient for an aqueous phase component, from the viewpoint of stability over time. Examples of the polyhydric alcohol include glycerin, 1,3-butandiol, and ethylene glycol; and polysaccharides such as reduced sugar syrup, sucrose, erythritol, xylitol, glucose, galactose, sorbitol, maltotriose, and trehalose. These may be used singly or in combinations of two or more thereof.

The content of the polyhydric alcohol in the oil-in-water cosmetic is preferably from 1 % by mass to 10 % by mass, and more preferably from 1 % by mass to 8 % by mass, with respect to the total mass of the cosmetic, from the viewpoint of feeling of use.

In the invention, the aqueous phase in the oil-in-water cosmetic means the total of water and ingredients added as an aqueous phase (ingredients for an aqueous phase) when the oil-in-water emulsified composition is produced.

The oil-in-water cosmetic according to the invention may include a water soluble polymer as an ingredient for the aqueous phase.

Here, as a water soluble polymer, any polymer may be used as long as it is dissolved to at least approximately 0.001 % by mass or more in water (25°C). In the invention, it is possible to stabilize the oil-in-water cosmetic by using a water soluble polymer. In the invention, the "water soluble polymer" refers to water soluble polymers that have been usually used in cosmetic compositions, and is not particularly restricted.

Examples of the water soluble polymer that may be used in the invention include polysaccharides such as pectin, kappa-carrageenan, locust bean gum, guar gum, hydroxypropyl guar gum, xanthan gum, karaya gum, tamarind seed polysaccharide, arabic gum, tragacanth gum, hyaluronic acid, sodium hyaluronate, sodium chondroitin sulfate, and dextrin; proteins having a molecular weight of more than 5000 such as casein, albumin, methylated collagen, hydrolyzed collagen, water soluble collagen and gelatin; synthesized polymers such as acrylic polymers (for example, carboxyvinyl polymer, sodium polyacrylate, polyethyl acrylate, and polyacrylamide), polyvinyl alcohol, polyethylene glycol, and a block copolymer of ethylene oxide and propylene oxide (polyoxyethylene type polymers such as polyethylene glycol 20,000, 40,000, and 60,000, and polyoxyethylene polyoxypropylene copolymer type polymer); water soluble cellulose derivatives such as hydroxyethyl cellulose and methyl cellulose; and combinations of two or more thereof. These may be synthesized products or natural products.

The total content of the water soluble polymer in the oil-in-water cosmetic in the invention is preferably less than 1 % by mass, and more preferably from 0.001 % by mass to 1% by mass, with respect to the total mass of the cosmetic, from the viewpoint of feeling of use. A total content of the water soluble polymer of less than 1 % by mass suppresses sticky feeling, and therefore is preferable.

In the oil-in-water cosmetic according to the invention, other additives that are usually used for the application may further be added, for example, moisturizing agents such as glycine betaine, urea, neutral amino acids, and basic amino acids; drugs such as allantoin; organic powder such as cellulose powder, nylon powder, crosslinked silicone powder, crosslinked methyl polysiloxane, porous cellulose powder, and porous nylon powder; inorganic powder such as anhydrous silica, zinc oxide, and titanium oxide; and algefacients such as menthol and camphor; plant extracts, pH buffers, antioxidants, ultraviolet absorbing agents, preservatives, fragrances, sanitizers, and coloring matters, to the extent that the effect of the invention is not impaired.

The oil-in-water cosmetic according to the invention can be produced by a general method of producing an emulsified composition, and specific examples thereof include an emulsifier-in-water method, an emulsifier-in-oil method, and an alternate addition method.

The oil-in-water cosmetic according to the invention may be used as, for example, a cosmetic for a skin care such as lotion, milky lotion, cream, eye cream, a serum, a massage preparation, a pack preparation, hand cream, or body cream, on a cosmetic for a makeup such as a makeup base. In particular, the oil-in-water cosmetic according to the invention may be preferably used as a cosmetic applied to a face, particularly around eyes.

### EXAMPLES

Hereinafter, the present invention is described in detail with reference to examples. However, the present invention is not limited thereto. Further, "%" is based on mass, unless otherwise specified.

### [Preparation of an oil-in-water cosmetic]

Creams as oil-in-water cosmetics of Examples 4, 9, 11, 13, 14, 15, 19, 24 of this invention, Reference Examples1-3, 5-8, 10, 12, 16-18, 20-23, 25, and Comparative Examples 1 to 9 were prepared according to formulations shown in Tables 1 to 7, by the following method. The values of each component in Tables 1 to 7 are indicated in % by mass.

According to the formulations shown in Tables 1 to 7, the preparation of oil phase components was carried out by mixing ingredients for oil phase components while heating at 80°C. Subsequently, according to the formulations shown in Tables 1 to 7, ingredients for an aqueous phase were mixed while heating at 80°C, the oil phase components prepared above were added thereto, and the mixture was emulsified using a homomixer. The emulsions obtained were cooled down to 30°C, thereby obtaining creams.

### [Evaluation Method]

### (1) Feeling of use (Resilient Feeling, Feeling of Persistence of Resilient Feeling, Moisturizing Feeling, and Feeling of Persistence of Moisturizing Feeling)

Each cream obtained above was applied to faces of a panel of 10 specialists, and according to the following criteria, resilient feeling and moisturizing feeling were evaluated each of: right after the application; and 6 hours after the application.
A: Evaluated as excellent by 8 to fewer than 10 people of the panel of 10 specialists
B: Evaluated as excellent by 6 to fewer than 8 people of the panel of 10 specialists
C: Evaluated as excellent by 4 to fewer than 6 people of the panel of 10 specialists
D: Evaluated as excellent by fewer than 4 people of the panel of 10 specialists

### (2) Stability

100g of each cream obtained above was weighed out and placed in a glass jar, and the jar was hermetically sealed and left to stand for a month under an environment of 40°C / 25°C / 4°C. After one month, stability over time was evaluated based on appearance, observation under an optical microscope, the result of the particle size distribution measurement, and the result of hardness measurement. The evaluation was made as follows.

LA-920 manufactured by HORIBA was used for the particle size distribution measurement, and the evaluation was made based on d50 (median size) the particle size distribution.
As to the hardness measurement, a rheometer FUDOH was used with respect to each cream in the glass jar obtained above, and the evaluation was made based on the change in hardness when a 20ϕ adaptor penetrates therein for 20mm.

Each of the evaluation results are shown in Tables 1 to 7.

(a) Appearance
   A: No separation is seen
   B: Very slight separation is seen
   C: Severe separation is seen
(b) Observation under Optical Microscope (×1050)
   A: Neither of generation of coarse particles due to aggregation nor coalescence, or precipitation of crystals or the like is found, and the appearance is equivalent to that just after the preparation
   B: Generation of coarse particles due to aggregation or coalescence or precipitation of crystals or the like is slightly found
   C: Generation of coarse particles due to aggregation or coalescence or precipitation of crystals or the like occurs in a large amount

(c) Particle Size Distribution Measurement
   A: The particle size distribution d50 is 0.5 µm or more but less than 2.0 µm
   B: The particle size distribution d50 is 2.0 µm or more but less than 3.0 µm
   C: The particle size distribution d50 is 3.0 µm or more but 4.0 µm or less
(d) Hardness Measurement
   A: Change in hardness as compared to the state just after the preparation is less than 5%
   B: Change in hardness as compared to the state just after the preparation is 5% or more but less than 15%
   C: Change in hardness as compared to the state just after the preparation is 15% or more but less than 35%
   D: Change in hardness as compared to the state just after the preparation is 35% or more.

In tables 1 to 5, RE means Reference Example, and IE means Inventive Example.

**[Table 1]**

| | | Examples | | | | | | | | | | | | | | |
|---|---|---|---|---|---|---|---|---|---|---|---|---|---|---|---|---|
| | | 1 | | | 2 | | | 3 | | | 4 | | | 5 | | |
| Oil Phase | Liquid Paraffin | 15 | | | 15 | | | 15 | | | 15 | | | 15 | | |
| | (A) Hydrogenated Polyisobutene, Kinematic viscosity: 300 mm²/s | - | | | - | | | - | | | - | | | - | | |
| | (A) Hydrogenated Polyisobutene, Kinematic viscosity: 800 mm²/s | 10 | | | 10 | | | 10 | | | 10 | | | 10 | | |
| | Hydrogenated Polyisobutene, Kinematic viscosity : 4700 mm²/s | - | | | - | | | - | | | - | | | - | | |
| | Vaseline | 11 | | | 11 | | | 11 | | | 11 | | | 11 | | |
| | Polysorbate 60 | 7 | | | 7 | | | 7 | | | 7 | | | 10 | | |
| | Glyceryl Stearate | 3 | | | 3 | | | 3 | | | 3 | | | 5 | | |
| | Stearic Acid | - | | | 2 | | | - | | | 2 | | | - | | |
| | Behenyl Alcohol | - | | | - | | | 2 | | | 2 | | | - | | |
| | (B) Sorbitan Stearate | 1 | | | 1 | | | 1 | | | 1 | | | 1 | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | 21.3 | | | 20.4 | | | 20.4 | | | 19.6 | | | 19.2 | | |
| | Amount of (A) Component relative to (B) Component (Times) | 10.0 | | | 10.0 | | | 10.0 | | | 10.0 | | | 10.0 | | |
| | Resilient Feeling | B | | | A | | | A | | | A | | | B | | |
| | Persistence of Resilient Feeling (After 6 hrs) | C | | | B | | | B | | | A | | | C | | |
| | Moisturizing Feeling | B | | | B | | | B | | | A | | | B | | |
| | Persistence of Moisturizing Feeling (After 6 hrs) | C | | | B | | | B | | | A | | | C | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | A | A | A | A | A | A |
| | Observation under Optical Microscope | A | A | A | A | A | A | A | A | A | A | A | A | A | A | A |
| | Particle Size Distribution Measurement | B | A | A | A | A | A | A | A | A | A | A | A | A | A | A |
| | Hardness Measurement | C | B | B | B | B | B | B | B | B | A | A | A | C | B | B |

**[Table 2]**

| | | Examples | | | | | | | | | | | | | | |
|---|---|---|---|---|---|---|---|---|---|---|---|---|---|---|---|---|
| | | 6 | | | 7 | | | 8 | | | 9 | | | 10 | | |
| Oil Phase | Liquid Paraffin | 15 | | | 15 | | | 15 | | | 15 | | | 15 | | |
| | (A) Hydrogenated Polyisobutene, Kinematic viscosity : 300 mm²/s | 12 | | | 12 | | | 12 | | | 12 | | | 12 | | |
| | (A) Hydrogenated Polyisobutene, Kinematic viscosity : 800 mm²/s | - | | | - | | | - | | | - | | | - | | |
| | Hydrogenated Polyisobutene, Kinematic viscosity : 4700 mm²/s | - | | | - | | | - | | | - | | | - | | |
| | Vaseline | 11 | | | 11 | | | 11 | | | 11 | | | 11 | | |
| | Polysorbate 60 | 7 | | | 7 | | | 7 | | | 7 | | | 10 | | |
| | Glyceryl Stearate | 3 | | | 3 | | | 3 | | | 3 | | | 5 | | |
| | Stearic Acid | - | | | 2 | | | - | | | 2 | | | - | | |
| | Behenyl Alcohol | - | | | - | | | 2 | | | 2 | | | - | | |
| | (B) Sorbitan Stearate | 1 | | | 1 | | | 1 | | | 1 | | | 1 | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate m Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | 24.5 | | | 23.5 | | | 23.5 | | | 22.6 | | | 22.2 | | |
| | Amount of (A) Component relative to (B) Component (Times) | 12.0 | | | 12.0 | | | 12.0 | | | 12.0 | | | 12.0 | | |
| | Resilient Feeling | B | | | A | | | A | | | A | | | B | | |
| | Persistence of Resilient Feeling (After 6 hrs) | C | | | C | | | C | | | A | | | C | | |
| | Moisturizing Feeling | B | | | B | | | B | | | A | | | B | | |
| | Persistence of Moisturizing Feeling (After 6 hrs) | C | | | C | | | C | | | A | | | C | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | A | A | A | A | A | A |
| | Observation under Optical Microscope | A | A | A | A | A | A | A | A | A | A | A | A | A | A | A |
| | Particle Size Distribution Measurement | B | A | A | A | A | A | A | A | A | A | A | A | A | A | A |
| | Hardness Measurement | C | B | B | B | B | B | B | B | B | A | A | A | C | B | B |

**[Table 3]**

| | | Examples | | | | | | | | | | | | | | |
|---|---|---|---|---|---|---|---|---|---|---|---|---|---|---|---|---|
| | | 11 | | | 12 | | | 13 | | | 14 | | | 15 | | |
| Oil Phase | Liquid Paraffin | 15 | | | 17 | | | 15 | | | 15 | | | 15 | | |
| | (A) Hydrogenated Polyisobutene, Kinematic viscosity: 300 mm²/s | - | | | - | | | - | | | - | | | - | | |
| | (A) Hydrogenated Polyisobutene, Kinematic viscosity: 800 mm²/s | 9 | | | 8 | | | 15 | | | 10 | | | 10 | | |
| | Hydrogenated Polyisobutene, Kinematic viscosity : 4700 mm²/s | - | | | - | | | - | | | - | | | - | | |
| | Vaseline | 10 | | | 13 | | | 6 | | | 11 | | | 11 | | |
| | Polysorbate 60 | 7 | | | 7 | | | 7 | | | 7 | | | 7 | | |
| | Glyceryl Stearate | 3 | | | 3 | | | 3 | | | 3 | | | 3 | | |
| | Stearic Acid | 2 | | | - | | | 2 | | | 2 | | | 2 | | |
| | Behenyl Alcohol | 2 | | | - | | | 2 | | | 2 | | | 2 | | |
| | (B) Sorbitan Stearate | 1 | | | 1 | | | 1 | | | 0.75 | | | 0.5 | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | 18.4 | | | 16.3 | | | 29.4 | | | 19.7 | | | 19.8 | | |
| | Amount of (A) Component relative to (B) Component (Times) | 9.0 | | | 8.0 | | | 15 | | | 13 | | | 20 | | |
| | Resilient Feeling | A | | | B | | | B | | | A | | | A | | |
| | Persistence of Resilient Feeling (After 6 hrs) | A | | | C | | | B | | | A | | | A | | |
| | Moisturizing Feeling | A | | | B | | | B | | | A | | | A | | |
| | Persistence of Moisturizing Feeling (After 6 hrs) | A | | | C | | | B | | | A | | | A | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | A | A | A | A | A | A |
| | Observation under Optical Microscope | A | A | A | A | A | A | A | A | A | A | A | A | A | A | A |
| | Particle Size Distribution Measurement | A | A | A | B | A | A | A | A | A | A | A | A | A | A | A |
| | Hardness Measurement | A | A | A | C | B | B | A | A | A | A | A | A | A | A | A |

**[Table 4]**

| | | Examples | | | | | | | | | | | | | | |
|---|---|---|---|---|---|---|---|---|---|---|---|---|---|---|---|---|
| | | 16 | | | 17 | | | 18 | | | 19 | | | 20 | | |
| Oil Phase | Liquid Paraffin | 15 | | | 15 | | | 15 | | | 15 | | | 15 | | |
| | (A) Hydrogenated Polyisobutene, Kinematic viscosity: 300 mm²/s | - | | | - | | | - | | | - | | | - | | |
| | (A) Hydrogenated Polyisobutene, Kinematic viscosity: 800 mm²/s | 10 | | | 10 | | | 10 | | | 10 | | | 10 | | |
| | Hydrogenated Polyisobutene, Kinematic viscosity : 4700 mm²/s | - | | | - | | | - | | | - | | | - | | |
| | Vaseline | 11 | | | 11 | | | 11 | | | 11 | | | 11 | | |
| | Polysorbate 60 | 7 | | | 7 | | | 7 | | | 7 | | | 10 | | |
| | Glyceryl Stearate | 3 | | | 3 | | | 3 | | | 3 | | | 5 | | |
| | Stearic Acid | - | | | 2 | | | - | | | 2 | | | - | | |
| | Behenyl Alcohol | - | | | - | | | 2 | | | 2 | | | - | | |
| | (B) Sorbitan Stearate | 1 | | | 1 | | | 1 | | | 1 | | | 1 | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | 21.3 | | | 20.4 | | | 20.4 | | | 19.6 | | | 19.2 | | |
| | Amount of (A) Component relative to (B) Component (Times) | 10.0 | | | 10.0 | | | 10.0 | | | 10.0 | | | 10.0 | | |
| | Resilient Feeling | B | | | A | | | A | | | A | | | B | | |
| | Persistence of Resilient Feeling (After 6 hrs) | C | | | B | | | B | | | A | | | C | | |
| | Moisturizing Feeling | B | | | B | | | B | | | A | | | B | | |
| | Persistence of Moisturizing Feeling (After 6 hrs) | C | | | B | | | B | | | A | | | C | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | A | A | A | A | A | A |
| | Observation under Optical Microscope | A | A | A | A | A | A | A | A | A | A | A | A | A | A | A |
| | Particle Size Distribution Measurement | B | A | A | A | A | A | A | A | A | A | A | A | A | A | A |
| | Hardness Measurement | C | B | B | B | B | B | B | B | B | A | A | A | C | B | B |

**[Table 5]**

| | | Examples | | | | | | | | | | | | | | |
|---|---|---|---|---|---|---|---|---|---|---|---|---|---|---|---|---|
| | | 21 | | | 22 | | | 23 | | | 24 | | | 25 | | |
| Oil Phase | Liquid Paraffin | 15 | | | 15 | | | 15 | | | 15 | | | 15 | | |
| | (A) Hydrogenated Polyisobutene, Kinematic viscosity : 300 mm²/s | - | | | - | | | - | | | - | | | - | | |
| | (A) Hydrogenated Polyisobutene, Kinematic viscosity : 800 mm²/s | 10 | | | 10 | | | 10 | | | 10 | | | 10 | | |
| | Hydrogenated Polyisobutene, Kinematic viscosity : 4700 mm²/s | - | | | - | | | - | | | - | | | - | | |
| | Vaseline | 11 | | | 11 | | | 11 | | | 11 | | | 11 | | |
| | Polysorbate 60 | 7 | | | 7 | | | 7 | | | 7 | | | 10 | | |
| | Glyceryl Stearate | 3 | | | 3 | | | 3 | | | 3 | | | 5 | | |
| | Stearic Acid | - | | | 2 | | | - | | | 2 | | | - | | |
| | Behenyl Alcohol | - | | | - | | | 2 | | | 2 | | | - | | |
| | (B) Sorbitan Stearate | 1 | | | 1 | | | 1 | | | 1 | | | 1 | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | 21.3 | | | 20.4 | | | 20.4 | | | 19.6 | | | 19.2 | | |
| | Amount of (A) Component relative to (B) Component (Times) | 10.0 | | | 10.0 | | | 10.0 | | | 10.0 | | | 10.0 | | |
| | Resilient Feeling | B | | | A | | | A | | | A | | | B | | |
| | Persistence of Resilient Feeling (After 6 hrs) | C | | | C | | | C | | | A | | | C | | |
| | Moisturizing Feeling | B | | | B | | | B | | | A | | | B | | |
| | Persistence of Moisturizing Feeling (After 6 hrs) | C | | | C | | | C | | | A | | | C | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | A | A | A | A | A | A |
| | Observation under Optical Microscope | A | A | A | A | A | A | A | A | A | A | A | A | A | A | A |
| | Particle Size Distribution Measurement | B | A | A | A | A | A | A | A | A | A | A | A | A | A | A |
| | Hardness Measurement | c | B | B | B | B | B | B | B | B | A | A | A | c | B | B |

**[Table 6]**

| | | Comparative Examples | | | | | | | | | | | | | | |
|---|---|---|---|---|---|---|---|---|---|---|---|---|---|---|---|---|
| | | 1 | | | 2 | | | 3 | | | 4 | | | 5 | | |
| Oil Phase | Liquid Paraffin | 15 | | | 15 | | | 15 | | | 15 | | | 20 | | |
| | (A) Hydrogenated Polyisobutene, Kinematic viscosity: 300 mm²/s | - | | | - | | | 10 | | | - | | | - | | |
| | (A) Hydrogenated Polyisobutene, Kinematic viscosity: 800 mm²/s | - | | | 10 | | | - | | | - | | | - | | |
| | Hydrogenated Polyisobutene, Kinematic viscosity: 4700 mm²/s | - | | | - | | | - | | | 10 | | | - | | |
| | Vaseline | 11 | | | 11 | | | 11 | | | 11 | | | 16 | | |
| | Polysorbate 60 | 7 | | | 7 | | | 7 | | | 7 | | | 7 | | |
| | Glyceryl Stearate | 3 | | | 3 | | | 3 | | | 3 | | | 3 | | |
| | Stearic Acid | - | | | - | | | - | | | - | | | - | | |
| | Stearyl Alcohol | - | | | - | | | - | | | - | | | - | | |
| | (B) Sorbitan Stearate | 1 | | | - | | | - | | | 1 | | | 1 | | |
| | Polyglyceryl Stearate-2 | - | | | - | | | - | | | - | | | - | | |
| | Sorbitan Oleate | - | | | - | | | - | | | - | | | - | | |
| Aqueous Phase | Glycerin | 8 | | | 8 | | | 8 | | | 8 | | | 8 | | |
| | Carboxy Vinyl Polymer | 0.2 | | | 0.2 | | | 0.2 | | | 0.2 | | | 0.2 | | |
| | KOH | 0.06 | | | 0.06 | | | 0.06 | | | 0.06 | | | 0.06 | | |
| | Preservative | Appropriate Amount | | | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | | Remaining Amount | | |
| | Quantitative Ratio of (A) Component relative to Oil Phase Component (%) | - | | | 21.7 | | | 21.7 | | | - | | | - | | |
| | Amount of (A) Component relative to (B) Component (Times) | | | | | | | | | | | | | | | |
| | Resilient Feeling | D | | | D | | | D | | | D | | | D | | |
| | Persistence of Resilient Feeling (After 6 hrs) | D | | | D | | | D | | | D | | | D | | |
| | Moisturizing Feeling | C | | | C | | | C | | | D | | | C | | |
| | Persistence of Moisturizing Feeling (After 6 hrs) | D | | | C | | | C | | | D | | | D | | |
| | Temperature at which cream was left to stand | 40°C | 25°C | 4°C | 40°C | 25°C | 4°C | 40°C | 25°C | 4°C | 40°C | 25°C | 4°C | 40°C | 25°C | 4°C |
| | Appearance | A | A | A | A | A | A | A | A | A | B | A | A | A | A | A |
| | Observation under Optical Microscope | A | A | A | A | A | A | A | A | A | B | A | A | A | A | A |
| | Particle Size Distribution Measurement | B | A | A | B | A | A | B | A | A | C | A | A | B | A | A |
| | Hardness Measurement | C | C | B | C | C | B | C | C | B | D | C | B | C | C | B |

**[Table 7]**

| | | Comparative Examples | | | | | | | | | | | |
|---|---|---|---|---|---|---|---|---|---|---|---|---|---|
| | | 6 | | | 7 | | | 8 | | | 9 | | |
| Oil Phase | Liquid Paraffin | 15 | | | 15 | | | 15 | | | 15 | | |
| | (A) Hydrogenated Polyisobutene, Kinematic viscosity; 300 mm²/s | - | | | - | | | - | | | - | | |
| | (A) Hydrogenated Polyisobutene, Kinematic viscosity: 800 mm²/s | - | | | - | | | 10 | | | 10 | | |
| | Hydrogenated Polyisobutene, Kinematic viscosity: 4700 mm²/s | - | | | 10 | | | - | | | - | | |
| | Vaseline | 21 | | | 11 | | | 11 | | | 11 | | |
| | Polysorbate 60 | 7 | | | 7 | | | 7 | | | 7 | | |
| | Glyceryl Stearate | 3 | | | 3 | | | 3 | | | 3 | | |
| | Stearic Acid | - | | | 2 | | | - | | | - | | |
| | Stearyl Alcohol | - | | | 2 | | | - | | | - | | |
| | (B) Sorbitan Stearate | 1 | | | 1 | | | - | | | - | | |
| | Polyglyceryl Stearate-2 | - | | | - | | | 1 | | | - | | |
| | Sorbitan Oleate | - | | | - | | | - | | | 1 | | |
| Aqueous Phase | Glycerin | 8 | | | 8 | | | 8 | | | 8 | | |
| | Carboxy Vinyl Polymer | 0.2 | | | 0.2 | | | 0.2 | | | 0.2 | | |
| | KOH | 0.06 | | | 0.06 | | | 0.06 | | | 0.06 | | |
| | Preservative | Appropriate Amount | | | Appropriate Amount | | | Appropriate Amount | | | Appropriate Amount | | |
| | Water | Remaining Amount | | | Remaining Amount | | | Remaining Amount | | | Remaining Amount | | |
| | Quantitative Ratio of (A) Component relative to Oil Phase Component (%) | - | | | - | | | 21.3 | | | 21.3 | | |
| | Amount of (A) Component relative to (B) Component (Times) | - | | | - | | | - | | | - | | |
| | Resilient Feeling | D | | | D | | | D | | | D | | |
| | Persistence of Resilient Feeling (After 6 hrs) | D | | | D | | | D | | | D | | |
| | Moisturizing Feeling | C | | | D | | | C | | | C | | |
| | Persistence of Moisturizing Feeling (After 6 hrs) | D | | | D | | | C | | | C | | |
| | Temperature at which cream was left to stand | 40°C | 25°C | 4°C | 40°C | 25°C | 4°C | 40°C | 25°C | 4°C | 40°C | 25°C | 4°C |
| | Appearance | A | A | A | B | A | A | A | A | A | A | A | A |
| | Observation under Optical Microscope | A | A | A | B | A | A | A | A | A | A | A | A |
| | Particle Size Distribution Measurement | B | A | A | C | A | A | B | A | A | B | A | A |
| | Hardness Measurement | C | C | A | D | C | B | C | C | B | C | C | B |

As shown in Tables 1 to 5, the creams which impart favorable resilient feeling without using a large amount of solid oil and which is stable without problems of separation and precipitation were successfully produced, the creams of Examples 4, 9, 11, 13, 14, 15, 19, 24 of this invention, and also of Reference Examples 1-3, 5-8, 10, 12, 16-18, 20-23, 25, in which the hydrogenated polyisobutene having a kinematic viscosity at 100 °C of from 200 mm²/s to 1000 mm²/s is combined with the sorbitan stearate.

It is known that the amount of a surfactant may be increased (Reference Examples 5, 10, 20, and 25) in order to increase the stability of an oil-in-water cosmetic. However, it is found that a method including at least one of a higher fatty acid or a higher alcohol is more effective (Reference Examples 2, 3, 7, 8, 17, 18, 22, 23 and Inventive Examples 4, 9.11, 13, 14, 15, 19 and 24).

In particular, in the case of cream including at least one of a higher fatty acid or a higher alcohol (Reference Examples 2, 3, 7, 8, 17, 18, 22, 23 and Inventive Examples 4, 9.11, 13, 14, 15, 19 and 24), a favorable result in terms of persistence of resilient feeling and moisturizing feeling in use was obtained, it is understood that the stability of the oil-in-water cosmetic is more favorable, and the function and the state of the oil-in-water cosmetic can persist for a long period of time.

In the present invention, both a higher fatty acid and a higher alcohol are used (Inventive Examples 4, 9, 11, 13, 14, 15, 19 and 24).

Generally, inclusion of polysorbate 60 is expected to exert an effect in terms of persistence of resilient feeling and moisturizing feeling. However, as indicated in Example 20, even when the amount of polysorbate 60 is increased as compared to Examples including a higher fatty acid and/or a higher alcohol, an effect in terms of persistence of resilient feeling and moisturizing feeling is not obtained. It is confirmed that inclusion of a higher fatty acid and a higher alcohol is more effective in terms of persistence of resilient feeling and moisturizing feeling in the present invention.

When either of a hydrogenated polyisobutene or sorbitan stearate is lacking (Comparative Examples 1 to 3), feeling of use as confirmed in Examples 4, 9, 11, 13, 14, 15, 19, 24 of this invention, and also of Reference Examples 1-3, 5-8, 10, 12, 16-18, 20-23, 25, in particular, resilient feeling, is not obtained at all.

Even when the amount of other oils such as liquid paraffin and vaseline is increased instead of the hydrogenated polyisobutene (Comparative Examples 5 and 6) or even when other surfactants are used instead of sorbitan stearate (Comparative Examples 8 and 9), resilient feeling equivalent to that in Examples 4, 9, 11, 13, 14, 15, 19, 24 of this invention, and also of Reference Examples 1-3, 5-8, 10, 12, 16-18, 20-23, 25 is not obtained. When a hydrogenated polyisobutene having outside the range of the kinematic viscosity at 100 °C of from 200 mm²/s to 1000 mm²/s is included (Comparative Examples 4 and 7), both feeling of use and stability deteriorated.

Accordingly, the oil-in-water cosmetic according to the invention has excellent stability over time, and is capable of imparting good feeling of use, in particular, resilient feeling.

## Claims

1. An oil-in-water cosmetic, comprising:
an aqueous phase; and
an oil phase including a hydrogenated polyisobutene having a kinematic viscosity at 100°C of from 200 mm²/s to 1000 mm²/s and sorbitan stearate,
wherein the kinematic viscosity is measured by the method described in JIS-K-2283, and
wherein the oil phase further comprises both a higher alcohol and a higher fatty acid, the higher alcohol being an alcohol having from 12 to 24 carbon atoms, and the higher fatty acid being a saturated or unsaturated fatty acid having from 12 to 24 carbon atoms.

2. The oil-in-water cosmetic according to claim 1, wherein a total content of a solid oil, which has a melting point of at least 60°C and is selected from an oil, a fat, a hydrocarbon or a wax, is 0 or less than 5% by mass, with respect to a total mass of the cosmetic.

3. The oil-in-water cosmetic according to claim 1 or 2, wherein a content of the oil phase is from 45% by mass to 70% by mass with respect to a total mass of the cosmetic.

4. The oil-in-water cosmetic according to any one of claims 1 to 3, wherein a content of the hydrogenated polyisobutene is from 10% by mass to 40% by mass with respect to the mass of the oil phase.

5. The oil-in-water cosmetic according to any one of claims 1 to 4, wherein a content of the hydrogenated polyisobutene is from 5% by mass to 20% by mass with respect to a total mass of the cosmetic.

6. The oil-in-water cosmetic according to any one of claims 1 to 5, wherein the higher alcohol is at least one selected from behenyl alcohol or stearyl alcohol.

7. The oil-in-water cosmetic according to any one of claims 1 to 6, wherein the higher fatty acid is at least one selected from palmitic acid or stearic acid.

## Patentansprüche

1. Öl-in-Wasser-Kosmetikum aufweisend:
eine wässrige Phase; und
eine Ölphase, die ein hydriertes Polyisobuten mit einer kinematischen Viskosität bei 100 °C von 200 mm²/s bis 1000 mm²/s und Sorbitanstearat enthält,
wobei die kinematische Viskosität mittels des in JIS-K-2283 beschriebenen Verfahrens gemessen wird, und
wobei die Ölphase außerdem sowohl einen höheren Alkohol als auch eine höhere Fettsäure aufweist, wobei der höhere Alkohol ein Alkohol mit 12 bis 24 Kohlenstoffatomen ist und die höhere Fettsäure eine gesättigte oder ungesättigte Fettsäure mit 12 bis 24 Kohlenstoffatomen ist.

2. Öl-in-Wasser-Kosmetikum nach Anspruch 1, bei dem der Gesamtgehalt an einem festen Öl, das einen Schmelzpunkt von mindestens 60 °C hat und ausgewählt ist aus einem Öl, einem Fett, einem Kohlenwasserstoff oder einem Wachs, 0 oder weniger als 5 Masseprozent, bezogen auf die Gesamtmasse des Kosmetikums, beträgt.

3. Öl-in-Wasser-Kosmetikum nach Anspruch 1 oder 2, bei dem der Gehalt der Ölphase 45 Masseprozent bis 70 Masseprozent, bezogen auf die Gesamtmasse des Kosmetikums, beträgt.

4. Öl-in-Wasser-Kosmetikum nach einem der Ansprüche 1 bis 3, bei dem der Gehalt an dem hydrierten Polyisobuten 10 Masseprozent bis 40 Masseprozent, bezogen auf die Masse der Ölphase, beträgt.

5. Öl-in-Wasser-Kosmetikum nach einem der Ansprüche 1 bis 4, bei dem der Gehalt an dem hydrierten Polyisobuten 5 Masseprozent bis 20 Masseprozent, bezogen auf die Gesamtmasse des Kosmetikums, beträgt.

6. Öl-in-Wasser-Kosmetikum nach einem der Ansprüche 1 bis 5, bei dem der höhere Alkohol mindestens ein Alkohol ist, der ausgewählt ist aus Behenylalkohol oder Stearylalkohol.

7. Öl-in-Wasser-Kosmetikum nach einem der Ansprüche 1 bis 6, bei dem die höhere Fettsäure mindestens eine höhere Fettsäure ist, die ausgewählt ist aus Palmitinsäure oder Stearinsäure.

## Revendications

1. Cosmétique huile dans l'eau, comprenant :
une phase aqueuse, et
une phase huileuse incluant un polyisobutène hydrogéné présentant une viscosité cinématique à 100 °C de 200 mm²/s à 1000 mm²/s et du stéarate de sorbitane,
dans lequel la viscosité cinématique est mesurée par la méthode décrite dans JIS-K-2283, et
dans lequel la phase huileuse comprend en outre à la fois un alcool supérieur et un acide gras supérieur, l'alcool supérieur étant un alcool présentant de 12 à 24 atomes de carbone, et l'acide gras supérieur étant un acide gras saturé ou insaturé présentant de 12 à 24 atomes de carbone.

2. Cosmétique huile dans l'eau selon la revendication 1, dans lequel une teneur totale d'une huile solide, laquelle présente un point de fusion d'au moins 60°C et est sélectionnée parmi une huile, une graisse, un hydrocarbure ou une cire, est égale à 0 ou inférieure à 5 % en masse, par rapport à une masse totale du cosmétique.

3. Cosmétique huile dans l'eau selon la revendication 1 ou 2, dans lequel une teneur de la phase huileuse s'étend de 45 % en masse à 70 % en masse, par rapport à une masse totale du cosmétique.

4. Cosmétique huile dans l'eau selon l'une quelconque des revendications 1 à 3, dans lequel une teneur du polyisobutène hydrogéné s'étend de 10 % en masse à 40 % en masse, par rapport à la masse de la phase huileuse.

5. Cosmétique huile dans l'eau selon l'une quelconque des revendications 1 à 4, dans lequel une teneur du polyisobutène hydrogéné s'étend de 5 % en masse à 20 % en masse, par rapport à une masse totale du cosmétique.

6. Cosmétique huile dans l'eau selon l'une quelconque des revendications 1 à 5, dans lequel l'alcool supérieur est au moins un élément sélectionné parmi un alcool béhénylique ou un alcool stéarique.

7. Cosmétique huile dans l'eau selon l'une quelconque des revendications 1 à 6, dans lequel l'acide gras supérieur est au moins un élément sélectionné parmi un acide palmitique ou un acide stéarique.
